# EUROPEAN PATENT SPECIFICATION

(11) **EP 3 278 678 B1**
(45) Date of publication and mention of the grant of the patent: **09.10.2019**
(21) Application number: 16885445.3
(22) Date of filing: 22.06.2016
(51) Int. Cl.: A24F 47/00

(54) **ELECTRONIC CIGARETTE ATOMIZER AND ELECTRONIC CIGARETTE**
ZERSTÄUBER FÜR ELEKTRONISCHE ZIGARETTE UND ELEKTRONISCHE ZIGARETTE
ATOMISEUR DE CIGARETTE ÉLECTRONIQUE ET CIGARETTE ÉLECTRONIQUE

(30) Priority: 03.06.2016 CN 201610390050
(43) Date of publication of application: 07.02.2018
(73) Proprietor: China Tobacco Hunan Industrial Co., Ltd., Changsha, Hunan 410007 (CN)
(72) Inventor: LIU, Jianfu, Changsha Hunan 410007 (CN); ZHONG, Kejun, Changsha Hunan 410007 (CN); GUO, Xiaoyi, Changsha Hunan 410007 (CN); HUANG, Wei, Changsha Hunan 410007 (CN); DAI, Yuangang, Changsha Hunan 410007 (CN); YIN, Xinqiang, Changsha Hunan 410007 (CN); YI, Jianhua, Changsha Hunan 410007 (CN); YU, Hong, Changsha Hunan 410007 (CN); ZHOU, Yongquan, Changsha Hunan 410007 (CN)
(74) Representative: Kramer Barske Schmidtchen Patentanwälte PartG mbB
(86) International application number: PCT/CN2016/086659
(87) International publication number: WO 2017/206212

(56) References cited:
- WO-A1-2015/128499
- CN-A- 103 919 279
- CN-A- 105 559 151
- CN-U- 204 466 914
- CN-U- 204 907 924
- CN-U- 205 233 465
- CN-U- 205 671 480
- JP-A- 2001 069 963

## Description

### Technical Field

The present invention relates to an atomizer of electronic cigarette and electronic cigarette.

### Background Art

The taste of the existing electronic cigarette has great difference with that of the cigarette on the market, thereby affecting the popularity and promotion of the electronic cigarette. Currently, common solution is to heat and atomize tobacco tar by using heating wires to produce smoke, but the taste of the smoke has a great difference with that of the real cigarette, thereby failing to meet users' demands; meanwhile the heating wires in the electronic cigarette require high power to atomize the tobacco tar to the smoke, thereby being likely to produce the burnt flavor, resulting in that the smoking taste becomes worse. Moreover, the structure of the existing electronic cigarette is complicated, and the tobacco tar is easy to leak. Thus, to meet the market demands, it is necessary to develop electronic cigarette that has the taste of the real cigarette to fill in the gap in the market and improve the user experience of the electronic cigarette.

CN103919279A discloses a hybrid e-cigarette with an ultrasonic atomizer for atomizing an e-liquid, and a separate heater for heating solid tobacco.

CN205233465U discloses an e-cigarette with an ultrasonic atomizer (piezo-electrical) for heating solid tobacco.

### Contents of Invention

The technical problem to be solved in the present invention is to provide an atomizer of electronic cigarette and electronic cigarette in view of the shortcomings of the prior art.

To solve the above-mentioned technical problem, the present invention adopts the technical solutions as follows: an atomizer of electronic cigarette comprises a housing; the housing is in fixed connection with a suction nozzle; an ultrasonic atomization piece is fixed in the housing; the ultrasonic atomization piece comprises a solid piezoelectric ceramic piece; one surface of the solid piezoelectric ceramic piece is in contact with a liquid guide structure, and the other surface thereof is in contact with solid perfume, so that the solid piezoelectric ceramic piece can not only atomize tobacco tar to produce smoke in a working process, but also transfer the heat generated by itself in the working process to the solid perfume, making the solid perfume to release fragrance; the liquid guide structure communicates with a tobacco tar storage cavity; both of the liquid guide structure and the solid perfume communicate with an airflow passage; and an air inlet of the airflow passage communicates with the outside of the housing, and an outlet of the airflow passage communicates with the suction nozzle, so that the smoke is mixed with the fragrance and is inhaled by a user through the suction nozzle.

In the present invention, the solid perfume comprises a cigarette material, a tobacco extract, essence and a humectant; and the weight ratio of the cigarette material, the tobacco extract, the essence and the humectant is 1: 0.1-0.5: 0.001-0.005: 2-6.

The plane where the solid piezoelectric ceramic piece is located is parallel to the center axis of the housing, therefore, when the atomizer of electronic cigarette is vertically placed on a horizontal plane, the radial direction of the solid piezoelectric ceramic piece is vertical to the horizontal plane, namely, the solid piezoelectric ceramic piece is vertically arranged in the housing, therefore the tobacco tar on the solid piezoelectric ceramic piece will not accumulate on the surface of the solid piezoelectric ceramic piece, and thus a higher starting speed of the atomizer can be guaranteed.

In a preferred technical solution of the present invention, the liquid guide structure comprises a liquid guide body and a liquid storage piece which is in contact with one surface of the solid piezoelectric ceramic piece; the liquid storage piece is in contact with one end of the liquid guide body; the other end of the liquid guide body stretches into the liquid storage cavity; the liquid guide body comprises a connecting part which has matched shape and size with those of the liquid storage piece and has opposite position to that of the same, and a liquid guide part stretching into the liquid storage cavity and connected with the connecting part; and at least one projection which is in contact with the liquid storage piece is provided on the connecting part. The projection props against the liquid storage piece to reserve a gap between the liquid guide body and the liquid storage piece, thereby ensuring that the airflow passing through the gap carries smoke and the airflow passage is smooth.

The connecting part is fixed in a fixing base, a notch is provided in the fixing base, and the liquid guide part extends into the liquid storage cavity from the notch. Therefore, the liquid guide part absorbs the tobacco tar in the liquid storage cavity and supplies the tobacco tar to the solid piezoelectric ceramic piece to be atomized.

Both of the ultrasonic atomization piece and the liquid storage piece are fixed in a bracket; the fixing base is connected with one side of the bracket which is close to the liquid storage piece; a perfume fixing base is provided on one side of the bracket which is away from the liquid storage piece; a cavity, namely a filling cavity, is provided between the perfume fixing base and the solid piezoelectric ceramic piece; the filling cavity is filled with the solid perfume; an air inlet hole and an air outlet hole, which are located at opposite positions and communicate with the solid perfume, are respectively provided in the bracket on the upper side and the lower side of the filling cavity; and the air outlet hole communicates with the suction nozzle, so that the airflow enters the filling cavity from the air inlet hole to discharge the fragrance in the filling cavity from the air outlet hole.

An air inlet is provided in the housing; a first gap is provided between the liquid storage piece and the liquid guide body, and one side of the first gap communicates with the air inlet; and the other side of the first gap communicates with the air inlet hole, so that the smoke produced by atomizing the tobacco tar by the solid piezoelectric ceramic piece is taken away by the airflow.

The liquid storage cavity is provided below the solid piezoelectric ceramic piece and the liquid guide body; a cavity cover is provided at the top end of the liquid storage cavity; one surface of the cavity cover which is away from the liquid storage cavity is connected with a side edge of a hollow cavity with an open upper end, and a limiting step is provided on the inner wall of the hollow cavity; the outer wall at the bottom of the bracket is connected with the inner wall of the limiting step; a first through hole is provided at a contact position of the bracket and the hollow cavity; the first gap communicates with the air inlet hole by the first through hole; and the air inlet, the first gap, the first through hole, the air inlet hole, the hollow cavity, the air outlet hole and the inner cavity of the suction nozzle form the airflow passage. Therefore, the airflow is carried into the filling cavity through the first gap to be mixed with the fragrance at first, making the taste of the smoke to be the same as the taste of real cigarette, and thus user's demands can be satisfied.

An installation through hole is provided in one side of the cavity cover; and the liquid guide part of the liquid guide body stretches into the liquid storage cavity after penetrating through the installation through hole. Therefore, the installation of the liquid guide body is more stable, and tobacco tar leakage is prevented.

The bottom end of the liquid storage cavity is provided in a bottom cover; and the outer wall of the bottom cover is in fixed connection with the inner wall of the housing, and the connection can be a snap connection or a magnetic connection so as to facilitate the detachment of the bottom cover.

In another preferred technical solution of the present invention, the liquid storage cavity is provided above the solid piezoelectric ceramic piece; the solid piezoelectric ceramic piece is fixed in a cavity, the cavity is formed by enclosing a lower fixing base which is open at both ends and an upper fixing base whose bottom end is provided in the lower fixing base; a vent groove is provided in the inner wall of the lower fixing base; the upper surface of the solid piezoelectric ceramic piece is in contact with the liquid guide structure; the lower surface of the solid piezoelectric ceramic piece is in contact with the solid perfume; and the lower fixing base is connected with the bottom of the housing; the liquid guide structure comprises a liquid guide body; the liquid guide body comprises a main body which is in contact with one surface of the solid piezoelectric ceramic piece; at least one projection is provided on the main body; and the projection communicates with the liquid storage cavity after penetrating through the upper fixing base. The structure is relatively simple, and the tobacco tar guide speed is relatively high.

A vent pipe is provided in the liquid storage cavity; an installation part is provided at the bottom end of the vent pipe; a mounting hole which has inside diameter matched with the outside diameter of the installation part is provided in the upper fixing base; the installation part is fixed in the mounting hole, and the inner cavity of the installation part communicates with the vent groove; and the top end of the vent pipe communicates with the inner cavity of the suction nozzle. Since the airflow passage is relatively long, the mixture of the smoke and the fragrance is more evenly, and thus the taste of the smoke is better.

The lower fixing base is provided on a connecting base; a second through hole is provided in the connecting base; a net structure is provided between the second through hole and the lower fixing base; the solid perfume is placed in the hollow cavity between the net structure and the solid piezoelectric ceramic piece, and the solid perfume is in contact with the solid piezoelectric ceramic piece, so that the heat produced by the solid piezoelectric ceramic piece in the working process is absorbed by the solid perfume, and the fragrance is released accordingly; and meanwhile, the net structure can not only fix the solid perfume, but also guarantee the airflow to be unobstructed.

An electrode is installed in the second through hole; an air inlet passage with one end communicated with the outside is provided in the electrode; the air inlet passage communicates with the vent groove through the net structure and the solid perfume; and the airflow passage is formed by the air inlet passage, the net structure, the solid perfume, the vent groove, the vent pipe and the inner cavity of the suction nozzle. Since the airflow passage is relatively long, the mixture of the smoke and the fragrance is more evenly, and thus the taste of the smoke is better.

At least one air hole communicated with the solid perfume is provided in the top of the electrode, and the air hole disperses the airflow, so that all the solid perfume is in contact with the airflow, and thus the fragrance can be taken away more easily.

Correspondingly, the present invention further provides an electronic cigarette, employing any atomizer of electronic cigarette in the above-mentioned two preferred technical solutions.

Compared with the prior art, the present invention has the following beneficial effects: the tobacco tar is atomized on one surface of the ultrasonic atomization piece in the present invention, when the tobacco tar is atomized, the ultrasonic atomization piece produces heat, and the other surface of the ultrasonic atomization piece transfers the heat to the solid perfume, so that the solid perfume releases fragrance when the ultrasonic atomization piece is working, the fragrance is mixed with the smoke produced by the atomization of the tobacco tar, therefore the taste of the electronic cigarette is closer to that of the cigarette, and the energy efficiency is high; that is to say, the ultrasonic atomization piece atomizes the tobacco tar to provide the smoke (tasteless smoke), the solid perfume provides the fragrance, and the smoke is mixed with the fragrance to form smoke having the taste and smell of cigarette; meanwhile, as one surface of the ultrasonic atomization piece is in contact with the tobacco tar in the liquid guide structure, the problem that the ultrasonic atomization piece is burnt in a dry state to be damaged easily is solved to a certain extent; and in the present invention, as the ultrasonic atomization piece atomizes the tobacco tar, the atomization power is low, no burnt flavor is produced, the structure is simple, exquisite, stable and compact, and promotion and application are easy to realize.

### Description of Figures

Fig. 1 is a sectional view of embodiment 1 of the present invention;
Fig. 2 is an explosive view of an atomization core in embodiment 1 of the present invention;
Fig. 3 is an explosive view of a liquid storage cavity and a bottom cover in the embodiment 1 of the present invention;
Fig. 4 is a sectional view of embodiment 2 of the present invention;
Fig. 5 is an explosive view of a structure between an upper fixing base and an electrode in embodiment 2 of the present invention;
Fig. 6 is an explosive view of a structure from a suction nozzle to the liquid storage cavity in embodiment 2 of the present invention.

### Specific Mode for Carrying Out the Invention

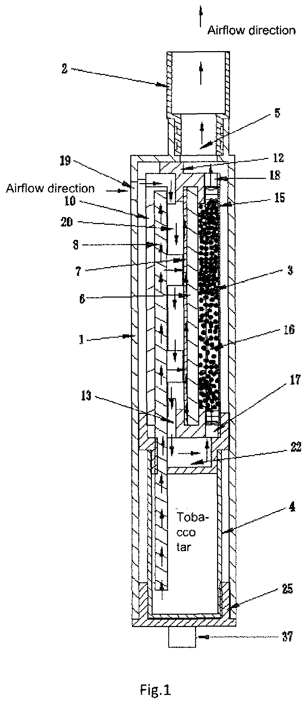

As shown in Fig. 1, embodiment 1 of the present invention comprises a housing 1; the housing 1 is in fixed connection with a suction nozzle 2; a solid piezoelectric ceramic piece 6 is fixed in the housing 1; one surface of the solid piezoelectric ceramic piece 6 is in contact with a liquid guide structure, and the other surface thereof is in contact with solid perfume 3; the liquid guide structure communicates with a tobacco tar storage cavity 4; both of the liquid guide structure and the solid perfume 3 communicate with an airflow passage 5; and an air inlet of the airflow passage 5 communicates with the outside of the housing 1, and an outlet of the airflow passage 5 communicates with the suction nozzle 2.

In the present invention, the solid perfume comprises a cigarette material, a tobacco extract, essence and a humectant, and solid perfume granules are prepared by warm air flow or convection heating or other processes and an automatic rolling mixer; and the weight ratio of the cigarette material, the tobacco extract, the essence and the humectant is 1: 0.1-0.5: 0.001-0.005: 2-6.

The cigarette material is one or more of cut tobacco, tobacco stem, tobacco slice and tobacco foam; the humectant is a mixture of propylene glycol and glycerol; and the essence is flavored perfume in tobacco flavor essence or strawberry flavor essence.

In the present invention, the ultrasonic atomization piece is a solid piezoelectric ceramic piece.

As shown in Fig. 1, the plane where the solid piezoelectric ceramic piece 6 is located is parallel to the center axis of the housing 1; that is to say, when the atomizer of electronic cigarette is vertically placed on a horizontal plane, the solid piezoelectric ceramic piece 6 is vertically placed in the atomizer of electronic cigarette; namely, the ultrasonic atomization piece is vertically provided in the housing 1.

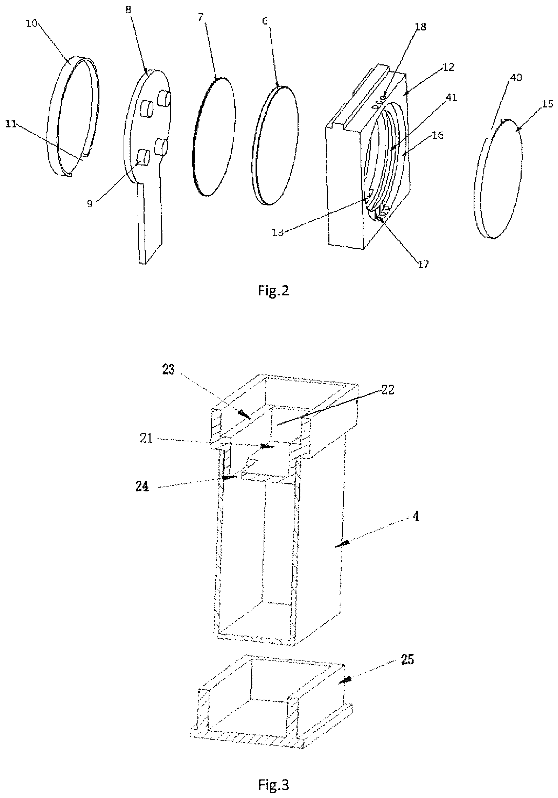

As shown in Fig. 2, the liquid guide structure comprises a liquid guide body 8 and a liquid storage piece 7 which is in contact with one surface of the solid piezoelectric ceramic piece 6; the liquid storage piece 7 is in contact with one end of the liquid guide body 8; the other end of the liquid guide body 8 stretches into the liquid storage cavity 4; the liquid guide body 8 comprises a connecting part which has matched shape and size with those of the liquid storage piece 7 and has opposite position to that of the same, and a liquid guide part stretching into the liquid storage cavity 4 and connected with the connecting part; and at least one projection 9 in contact with the liquid storage piece 7 is provided on the connecting part.

The connecting part is fixed in a fixing base 10, a notch 11 is provided in the fixing base 10, and the liquid guide part extends into the liquid storage cavity 4 from the notch 11. The fixing base 10 is of a disc-shaped structure, and the inner side of a side edge of the disc-shaped structure is in contact with the edge of the connecting part of the liquid guide body.

The liquid guide part of the liquid guide body is of a strip-shaped structure, the strip-shaped structure penetrates through the notch 11, and the width of the notch 11 is matched with the width of the strip-shaped structure.

As shown in Fig. 2, in the present invention, both of the solid piezoelectric ceramic piece 6 and the liquid storage piece 7 are fixed in a bracket 12; the fixing base 10 is connected with one side of the bracket 12 which is close to the liquid storage piece 7; a perfume fixing base 15 is provided on one side of the bracket 12 which is away from the liquid storage piece 7; a cavity, namely a filling cavity 16, is provided between the perfume fixing base 15 and the solid piezoelectric ceramic piece 6; the filling cavity 16 is filled with the solid perfume 3; an air inlet hole 17 and an air outlet hole 18, which are located at opposite positions and communicate with the solid perfume 3, are respectively provided in the bracket 12 on the upper side and the lower side of the filling cavity 16; and the air outlet hole 18 communicates with the suction nozzle 2.

The perfume fixing base 15 is of a disc-shaped structure, and corresponding side edges of the disc-shaped structure located on the air inlet hole 17 and the air outlet hole 18 are provided with notches 40, thereby ensuring that the airflow can enter into the filling cavity 16 through the air inlet hole 17 and can flow out from the air outlet hole 18 in the filling cavity 16.

An installation groove 41 is provided in the bracket 12, and the solid piezoelectric ceramic piece 6 and the liquid storage piece 7 are both fixed in the installation groove 41.

As shown in Fig. 1, an air inlet 19 is provided in the housing 1; the air inlet 19 communicates with a first gap 20 between the liquid storage piece 7 and the liquid guide body 8; the first gap 20 communicates with the air inlet hole 17; and the air inlet 19, the first gap 20, the air inlet hole 17, the air outlet hole 18 and the inner cavity of the suction nozzle 2 form the airflow passage 5. The smoke produced by the oscillation of the solid piezoelectric ceramic piece 6 is ejected from the surface of the liquid storage piece 7 and enters the first gap 20, and then the smoke enters the solid perfume through the air inlet hole 17 and is mixed with the fragrance produced by the solid perfume during the oscillation of the solid piezoelectric ceramic piece 6, and then the mixed smoke enters the suction nozzle through the air outlet hole 18 and is inhaled by the user.

As shown in Fig. 3, the liquid storage cavity 4 is provided below the solid piezoelectric ceramic piece 6 and the liquid guide body 8; a cavity cover 21 is provided at the top end of the liquid storage cavity 4; one surface of the cavity cover 21 which is away from the liquid storage cavity 4 is connected with a side edge of a hollow cavity 22 which has an open upper end, and a limiting step 23 is provided on the inner wall of the hollow cavity 22; the bottom of the bracket 12 props against the limiting step 23, and a first through hole 13 is provided at a place on the bracket 12 where the bracket 12 communicates with the hollow cavity 22; and the first through hole 13 communicates the first gap 20 with the air inlet hole 17.

An installation through hole 24 is provided in one side of the cavity cover 21; and the liquid guide part of the liquid guide body 8 stretches into the liquid storage cavity 4 after penetrating through the installation through hole 24 to absorb the tobacco tar so as to provide the tobacco tar for the atomization of the solid piezoelectric ceramic piece 6.

As shown in Fig. 3, the bottom end of the liquid storage cavity 4 is provided in a bottom cover 25; and the outer wall of the bottom cover 25 is in a snap connection or a magnetic connection with the inner wall of the housing 1.

An electrode 37 is fixed to a base 25 to facilitate the electric connection with a power supply component.

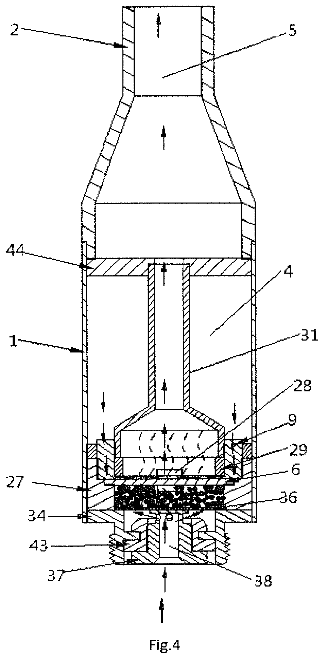

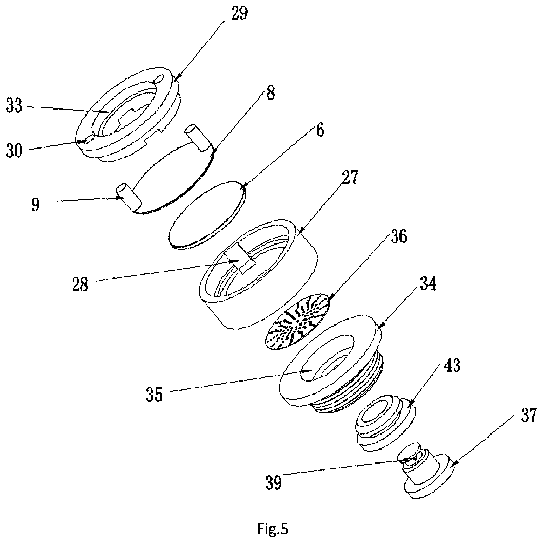

As shown in Fig. 4 and Fig. 5, in embodiment 2, the liquid storage cavity 4 is provided above the solid piezoelectric ceramic piece 6; the solid piezoelectric ceramic piece 6 is fixed in a cavity, the cavity is formed by enclosing a lower fixing base 27 which is open at both ends and an upper fixing base 29 whose bottom end is provided in the lower fixing base 27 together; a vent groove 28 is provided in the inner wall of the lower fixing base 27; the upper surface of the solid piezoelectric ceramic piece 6 is in contact with the liquid guide structure; the lower surface of the solid piezoelectric ceramic piece 6 is in contact with the solid perfume 3; and the lower fixing base 27 is connected with the bottom of the housing 1.

The liquid guide structure comprises a liquid guide body 8; the liquid guide body 8 comprises a main body whose diameter is greater than that of the solid piezoelectric ceramic piece 6 (facilitating the connection with the upper fixing base), and the main body has a circular bottom; at least one projection 9 is provided on the edge of the circular bottom; and the projection 9 communicates with the liquid storage cavity 4 after penetrating through a tobacco tar inlet hole 30 in the upper fixing base 29.

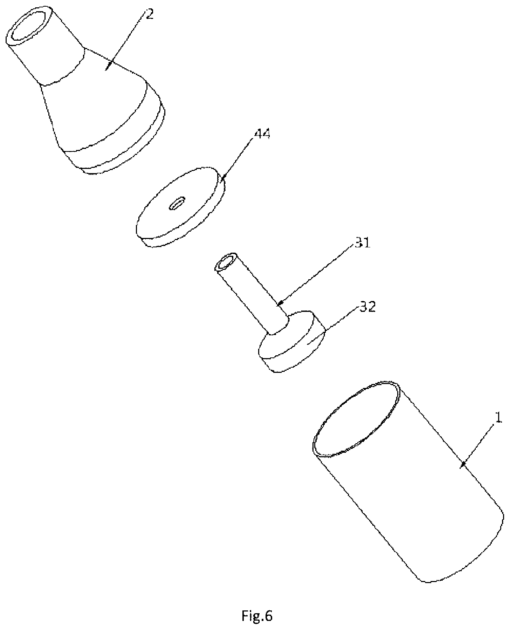

As shown in Fig. 4, Fig. 5 and Fig. 6, a vent pipe 31 is provided in the liquid storage cavity 4; an installation part 32 is provided at the bottom end of the vent pipe 31; a mounting hole 33 with inside diameter matched with the outside diameter of the installation part 32 is provided in the upper fixing base 29; the installation part 32 is fixed in the mounting hole 33, and the inner cavity of the installation part 32 communicates with the vent groove 28; and the top end of the vent pipe 31 communicates with the inner cavity of the suction nozzle 2.

The lower fixing base 27 is provided on a connecting base 34; a second through hole 35 is provided in the connecting base 34; a net structure 36 is provided between the second through hole 35 and the lower fixing base 27; the solid perfume 3 is placed in the hollow cavity between the net structure 36 and the solid piezoelectric ceramic piece 6, and the solid perfume 3 is in contact with the solid piezoelectric ceramic piece 6, so that the heat produced by the solid piezoelectric ceramic piece 6 in the working process is absorbed by the solid perfume 3, and the fragrance is released accordingly.

In the present invention, the net structure is a steel mesh.

An electrode 37 is installed in the second through hole 35 (the electrode 37 and the second through hole 35 are insulated and isolated by an insulation ring 43); an air inlet passage 38 with one end communicated with the outside is provided in the electrode 37; an air hole 39 communicated with the air inlet passage 38 is provided in the other end; and the air inlet passage 38 communicates with the vent groove 28 through a radial trough hole, the net structure 36 and the solid perfume 3; and the air inlet passage 38, the net structure 36, the solid perfume 3, the vent groove 28, the vent pipe 31 and the inner cavity of the suction nozzle 2 form the airflow passage 5.

External air enters from the air inlet passage 38 and flows into the solid perfume through the air hole 39 and the top end of the electrode 37, and then enters the vent pipe after being mixed with the smoke in the vent groove 28, wherein the smoke is produced by atomizing the tobacco tar by the solid piezoelectric ceramic piece 6, and finally enters the suction nozzle to be inhaled by the user.

In order to prevent the leakage of the tobacco tar of the atomizer, the connecting place of the housing 1 and the suction nozzle 2 is sealed by a sealing ring 44. Electronic cigarette of the present invention employs the atomizer of electronic cigarette of the embodiment 1 or the embodiment 2 mentioned above.

## Claims

1. An atomizer of electronic cigarette, comprising a housing (1) which is in fixed connection with a suction nozzle (2), wherein an ultrasonic atomization piece is fixed in the housing (1); the ultrasonic atomization piece comprises a solid piezoelectric ceramic piece (6); **characterized in that** one surface of the solid piezoelectric ceramic piece (6) is in contact with a liquid guide structure, and the other surface thereof is in contact with solid perfume (3); the liquid guide structure communicates with a tobacco tar storage cavity (4); both of the liquid guide structure and the solid perfume (3) communicate with an airflow passage (5); an air inlet of the airflow passage (5) communicates with the outside of the housing (1), and an outlet of the airflow passage (5) communicates with the suction nozzle (2).

2. The atomizer of electronic cigarette of claim 1, wherein the solid perfume comprises a cigarette material, a tobacco extract, essence and a humectant; and the weight ratio of the cigarette material, the tobacco extract, the essence and the humectant is 1: 0.1-0.5: 0.001-0.005: 2-6.

3. The atomizer of electronic cigarette of claim 1, wherein the plane where the solid piezoelectric ceramic piece (6) is located is parallel to the center axis of the housing (1).

4. The atomizer of electronic cigarette of claim 3, wherein the liquid guide structure comprises a liquid guide body (8) and a liquid storage piece (7) which is in contact with one surface of the solid piezoelectric ceramic piece (6); the liquid storage piece (7) is in contact with one end of the liquid guide body (8); and the other end of the liquid guide body (8) stretches into the liquid storage cavity (4).

5. The atomizer of electronic cigarette of claim 4, wherein the liquid guide body (8) comprises a connecting part which has matched shape and size with those of the liquid storage piece (7) and has opposite position to that of the same, and a liquid guide part stretching into the liquid storage cavity (4) and connected with the connecting part; and at least one projection (9) which is in contact with the liquid storage piece (7) is provided on the connecting part.

6. The atomizer of electronic cigarette of claim 5, wherein the connecting part is fixed in a fixing base (10), a notch (11) is provided in the fixing base (10), and the liquid guide part extends into the liquid storage cavity (4) from the notch (11).

7. The atomizer of electronic cigarette of claim 6, wherein both of the solid piezoelectric ceramic piece (6) and the liquid storage piece (7) are fixed in a bracket (12); the fixing base (10) is connected with one side of the bracket (12) which is close to the liquid storage piece (7); a perfume fixing base (15) is provided on one side of the bracket (12) which is away from the liquid storage piece (7); a cavity, namely a filling cavity (16) is provided between the perfume fixing base (15) and the solid piezoelectric ceramic piece (6); the filling cavity (16) is filled with the solid perfume (3); an air inlet hole (17) and an air outlet hole (18), which are located at opposite positions and communicate with the solid perfume (3), are respectively provided in the bracket (12) on the upper side and the lower side of the filling cavity (16); and the air outlet hole (18) communicates with the suction nozzle (2).

8. The atomizer of electronic cigarette of claim 7, wherein an air inlet (19) is provided in the housing (1); a first gap (20) is provided between the liquid storage piece (7) and the liquid guide body (8), and one side of the first gap (20) communicates with the air inlet (19); and the other side of the first gap (20) communicates with the air inlet hole (17).

9. The atomizer of electronic cigarette of claim 8, wherein the liquid storage cavity (4) is provided below the solid piezoelectric ceramic piece (6) and the liquid guide body (8); a cavity cover (21) is provided at the top end of the liquid storage cavity (4); one surface of the cavity cover (21) which is away from the liquid storage cavity (4) is connected with a side edge of a hollow cavity (22) which has an open upper end, and a limiting step (23) is provided on the inner wall of the hollow cavity (22); the outer wall at the bottom of the bracket (12) is connected with the inner wall of the limiting step (23); a first through hole (13) is provided at a contact position of the bracket (12) and the hollow cavity (22); the first gap (20) communicates with the air inlet hole (17) by the first through hole (13); and the air inlet (19), the first gap (20), the first through hole (13), the air inlet hole (17), the hollow cavity (22), the air outlet hole (18) and the inner cavity of the suction nozzle (2) form the airflow passage (5).

10. The atomizer of electronic cigarette of claim 9, wherein an installation through hole (24) is provided in one side of the cavity cover (21); and the liquid guide part of the liquid guide body (8) stretches into the liquid storage cavity (4) after penetrating through the installation through hole (24).

11. The atomizer of electronic cigarette of claim 9, wherein the bottom end of the liquid storage cavity (4) is provided in a bottom cover (25); and the outer wall of the bottom cover (25) is in fixed connection with the inner wall of the housing (1).

12. The atomizer of electronic cigarette of claim 1, wherein the liquid storage cavity (4) is provided above the solid piezoelectric ceramic piece (6); the solid piezoelectric ceramic piece (6) is fixed in a cavity, the cavity is formed by enclosing a lower fixing base (27) which is open at both ends and an upper fixing base (29) whose bottom end is provided in the lower fixing base (27); a vent groove (28) is provided in the inner wall of the lower fixing base (27); the upper surface of the solid piezoelectric ceramic piece (6) is in contact with the liquid guide structure; the lower surface of the solid piezoelectric ceramic piece (6) is in contact with the solid perfume (3); and the lower fixing base (27) is connected with the bottom of the housing (1).

13. The atomizer of electronic cigarette of claim 12, wherein the liquid guide structure comprises a liquid guide body (8); the liquid guide body (8) comprises a main body which is in contact with one surface of the solid piezoelectric ceramic piece (6); at least one projection (9) is provided on the main body; and the projection (9) communicates with the liquid storage cavity (4) after penetrating through the upper fixing base (29).

14. The atomizer of electronic cigarette of claim 13, wherein a vent pipe (31) is provided in the liquid storage cavity (4); an installation part (32) is provided at the bottom end of the vent pipe (31); a mounting hole (33) which has inside diameter matched with the outside diameter of the installation part (32) is provided in the upper fixing base (29); the installation part (32) is fixed in the mounting hole (33), and the inner cavity of the installation part (32) communicates with the vent groove (28); and the top end of the vent pipe (31) communicates with the inner cavity of the suction nozzle (2).

15. The atomizer of electronic cigarette of claim 14, wherein the lower fixing base (27) is provided on a connecting base (34); a second through hole (35) is provided in the connecting base (34); a net structure (36) is provided between the second through hole (35) and the lower fixing base (27); the solid perfume (3) is placed in the hollow cavity between the net structure (36) and the solid piezoelectric ceramic piece (6).

16. The atomizer of electronic cigarette of claim 15, wherein an electrode (37) is installed in the second through hole (35); an air inlet passage (38) with one end communicated with the outside is provided in the electrode (37); and the air inlet passage (38) communicates with the vent groove (28) through the net structure (36) and the solid perfume (3).

17. The atomizer of electronic cigarette of claim 16, wherein at least one air hole (39) communicated with the solid perfume (3) is provided in the top of the electrode (37); and the airflow passage (5) is formed by the air inlet passage (38), the air hole (39), the net structure (36), the solid perfume (3), the vent groove (28), the vent pipe (31) and the inner cavity of the suction nozzle (2).

18. An electronic cigarette, wherein the electronic cigarette employs the atomizer of electronic cigarette of any one of claims 1-17.

## Patentansprüche

1. Zerstäuber für eine elektrische Zigarette, mit einem Gehäuse (1), das in fester Verbindung mit einem Ansaugstutzen (2) steht, wobei ein Ultraschallzerstäubungsstück in dem Gehäuse (1) befestigt ist, das Ultraschallzerstäubungsstück ein festes piezoelektrisches Keramikstück (6) aufweist, **dadurch gekennzeichnet, dass**
eine Oberfläche des festen piezoelektrischen Keramikstücks (6) in Kontakt mit einer Flüssigkeitsführungsstruktur ist, und die andere Oberfläche desselben in Kontakt mit einem festen Duftstoff (3) ist, die Flüssigkeitsführungsstruktur mit einem Tabakteerspeicherhohlraum (4) in Verbindung steht, sowohl die Flüssigkeitsführungsstruktur (5) als auch der feste Duftstoff (3) mit einer Luftströmungspassage (5) in Verbindung stehen, ein Lufteinlass der Luftströmungspassage (5) mit der Außenseite des Gehäuses (1) in Verbindung steht, und ein Auslass der Luftströmungspassage (5) mit dem Ansaugstutzen (2) in Verbindung steht.

2. Zerstäuber für eine elektrische Zigarette nach Anspruch 1, bei dem der feste Duftstoff ein Zigarettenmaterial, ein Tabakextrakt, eine Essenz und ein Feuchthaltemittel umfasst, und das Gewichtsverhältnis des Zigarettenmaterials, des Tabakextrakts, der Essenz und des Feuchthaltemittels 1:0,1-0,5:0,001-0,005:2-6 beträgt.

3. Zerstäuber für eine elektrische Zigarette nach Anspruch 1, bei dem die Ebene, in welcher sich das feste piezoelektrische Keramikstück (6) befindet, parallel zu der Mittelachse des Gehäuses (1) verläuft.

4. Zerstäuber für eine elektrische Zigarette nach Anspruch 3, bei dem die Flüssigkeitsführungsstruktur einen Flüssigkeitsführungskörper (8) und ein Flüssigkeitsspeicherstück (7) aufweist, das in Kontakt mit der einen Oberfläche des festen piezoelektrischen Keramikstücks (6) ist, das Flüssigkeitsspeicherstück (7) in Kontakt mit einem Ende des Flüssigkeitsführungskörpers (8) ist, und das andere Ende des Flüssigkeitsführungskörpers (8) sich in den Flüssigkeitsspeicherhohlraum (4) erstreckt.

5. Zerstäuber für eine elektrische Zigarette nach Anspruch 4, bei dem der Flüssigkeitsführungskörper (8) einen Verbindungsteil, der eine passende Form und Größe mit denen des Flüssigkeitsspeicherstücks (7) aufweist und eine entgegengesetzte Position zu der des gleichen aufweist, und einen Flüssigkeitsführungsteil aufweist, der sich in den Flüssigkeitsspeicherhohlraum (4) erstreckt und mit dem Verbindungsteil verbunden ist, und zumindest ein Vorsprung (9), welcher in Kontakt mit dem Flüssigkeitsspeicherstück (7) ist, ist an dem Verbindungsteil vorgesehen.

6. Zerstäuber für eine elektrische Zigarette nach Anspruch 5, bei dem der Verbindungsteil in einer Fixierungsbasis (10) fixiert ist, eine Kerbe (11) in der Fixierungsbasis (10) vorgesehen ist, und der Flüssigkeitsführungsteil sich in den Flüssigkeitsspeicherhohlraum (4) von der Kerbe (11) erstreckt.

7. Zerstäuber für eine elektrische Zigarette nach Anspruch 6, bei dem sowohl das feste piezoelektrische Keramikstück (6) und das Flüssigkeitsspeicherstück (7) in einer Halterung (12) fixiert sind, die Fixierungsbasis (10) mit einer Seite der Halterung (12) verbunden ist, welche nahe zu dem Flüssigkeitsspeicherungsstück (7) ist, eine Duftstofffixierungsbasis (15) an einer Seite der Halterung (12) vorgesehen ist, welche entfernt von dem Flüssigkeitsspeicherstück (7) ist, ein Hohlraum, nämlich ein Füllhohlraum (16), zwischen der Duftstofffixierungsbasis (15) und dem festen piezoelektrischen Keramikstück (6) vorgesehen ist, der Füllhohlraum (16) mit dem festen Duftstoff (3) gefüllt ist, ein Lufteinlassloch (17) und ein Luftauslassloch (18), welche sich an entgegengesetzten Positionen befinden und mit dem festen Duftstoff (3) in Verbindung stehen, jeweils in der Halterung (12) an der oberen Seite und der unteren Seite des Füllhohlraums (16) vorgesehen sind, und das Luftauslassloch (18) mit dem Ansaugstutzen (2) in Verbindung steht.

8. Zerstäuber für eine elektrische Zigarette nach Anspruch 7, bei dem ein Lufteinlass (19) in dem Gehäuse (1) vorgesehen ist, ein erster Spalt (20) zwischen dem Flüssigkeitsspeicherstück (7) und dem Flüssigkeitsführungskörper (8) vorgesehen ist, und eine Seite des ersten Spalts (20) mit dem Lufteinlass (19) in Verbindung steht, und die andere Seite des ersten Spalts (20) mit dem Lufteinlassloch (17) in Verbindung steht.

9. Zerstäuber für eine elektrische Zigarette nach Anspruch 8, bei der der Flüssigkeitsspeicherhohlraum (4) unterhalb des festen piezoelektrischen Keramikstücks (6) und dem Flüssigkeitsführungskörper (8) vorgesehen ist, eine Hohlraumabdeckung (21) an dem oberen Ende des Flüssigkeitsspeicherhohlraums (4) vorgesehen ist, eine Oberfläche der Hohlraumabdeckung (21), welche entfernt von dem Flüssigkeitsspeicherhohlraum (4) ist, mit einer Seitenkante eines hohlen Hohlraums (22) verbunden ist, welcher ein offenes oberes Ende aufweist, und eine Begrenzungsstufe (23) an der Innenwand des hohlen Hohlraums (22) vorgesehen ist, die Außenwand an dem Boden der Halterung (12) mit der Innenwand der Begrenzungsstufe (23) verbunden ist, ein erstes Durchgangsloch (13) an einer Kontaktposition der Halterung (12) und dem hohlen Hohlraum (22) vorgesehen ist, der erste Spalt (20) mit einem Lufteinlassloch (17) bei dem ersten Durchgangsloch (13) in Verbindung steht, und der Lufteinlass (19), der erste Spalt (20), das erste Durchgangsloch (13), das Lufteinlassloch (17), der hohle Hohlraum (22), das Luftauslassloch (18) und der innere Hohlraum des Ansaugstutzens (2) die Luftströmungspassage (5) ausbilden.

10. Zerstäuber für eine elektrische Zigarette nach Anspruch 9, bei dem ein Installationsdurchgangsloch (24) an einer Seite der Hohlraumabdeckung (21) vorgesehen ist, und der Flüssigkeitsführungsteil des Flüssigkeitsführungskörpers (8) sich in den Flüssigkeitsspeicherhohlraum (4) nach Durchdringen durch das Installationsdurchgangsloch (4) erstreckt.

11. Zerstäuber für eine elektrische Zigarette nach Anspruch 9, bei dem das Bodenende des Flüssigkeitsspeicherhohlraums (4) in einer Bodenabdeckung (25) vorgesehen ist, und die Außenwand der Bodenabdeckung (25) in fester Verbindung mit der Innenwand des Gehäuses 1 ist.

12. Zerstäuber für eine elektrische Zigarette nach Anspruch 1, bei der der Flüssigkeitsspeicherhohlraum (4) oberhalb des festen piezoelektrischen Keramikstücks (6) vorgesehen ist, das feste piezoelektrische Keramikstück (6) in einem Hohlraum fixiert ist, der Hohlraum ausgebildet ist durch Umschließen einer unteren Fixierungsbasis (27), welche an beiden Enden geöffnet ist, und einer oberen Fixierungsbasis (29), deren Bodenende in der unteren Fixierungsbasis (27) vorgesehen ist, eine Entlüftungsnut (28) in der Innenwand der unteren Fixierungsbasis (27) vorgesehen ist, die obere Oberfläche des festen piezoelektrischen Keramikstücks (6) in Kontakt mit der Flüssigkeitsführungsstruktur ist, die untere Oberfläche des festen piezoelektrischen Keramikstücks (6) in Kontakt mit dem festen Duftstoff (3) ist, und die untere Fixierungsbasis (27) mit dem Boden des Gehäuses (1) verbunden ist.

13. Zerstäuber für eine elektrische Zigarette nach Anspruch 12, bei dem die Flüssigkeitsführungsstruktur einen Flüssigkeitsführungskörper (8) aufweist, der Flüssigkeitsführungskörper (8) einen Hauptkörper aufweist, der in Kontakt mit einer Oberfläche des festen piezoelektrischen Keramikstücks (6) ist, zumindest ein Vorsprung (9) an dem Hauptkörper vorgesehen ist, und der Vorsprung (9) mit dem Flüssigkeitsspeicherhohlraum (4) nach Durchdringen durch die obere Fixierungsbasis (29) in Verbindung steht.

14. Zerstäuber für eine elektrische Zigarette nach Anspruch 13, bei dem ein Entlüftungsrohr (31) in dem Flüssigkeitsspeicherhohlraum (4) vorgesehen ist, ein Installationsteil (32) an dem Bodenende des Entlüftungsrohrs (31) vorgesehen ist, ein Montageloch (33), welches einen Innendurchmesser aufweist, der passend mit dem Außendurchmesser des Installationsteils (32) ist, in der oberen Fixierungsbasis (29) vorgesehen ist, der Installationsteil (32) in dem Montageloch (33) fixiert ist, und der innere Hohlraum des Installationsteils (32) mit der Entlüftungsnut (82) in Verbindung steht, und das obere Ende des Entlüftungsrohrs (31) mit dem inneren Hohlraum des Ansaugstutzens (2) in Verbindung steht.

15. Zerstäuber für eine elektrische Zigarette nach Anspruch 14, bei dem die untere Fixierungsbasis (27) an einer Verbindungsbasis (34) vorgesehen ist, ein zweites Durchgangsloch (35) in der Verbindungsbasis (34) vorgesehen ist, eine Netzstruktur (36) zwischen dem zweiten Durchgangsloch (35) und der unteren Fixierungsbasis (27) vorgesehen ist, und der feste Duftstoff (3) in dem hohlen Hohlraum zwischen der Netzstruktur (36) und dem festen piezoelektrischen Keramikstück (6) platziert ist.

16. Zerstäuber für eine elektrische Zigarette nach Anspruch 15, bei dem eine Elektrode (37) in dem zweiten Durchgangsloch (35) installiert ist, eine Lufteinlasspassage (38), die mit einem Ende mit der Außenseite verbunden ist, in der Elektrode (37) vorgesehen ist, und die Lufteinlasspassage (38) mit der Entlüftungsnut (28) durch die Netzstruktur (36) und den festen Duftstoff (3) in Verbindung steht.

17. Zerstäuber für eine elektrische Zigarette nach Anspruch 16, bei dem zumindest ein Luftloch (39), das mit dem festen Duftstoff (3) in Verbindung steht, an der Oberseite der Elektrode (37) vorgesehen ist, und die Luftströmungspassage (5) durch die Lufteinlasspassage (38), dem Luftloch (39), der Netzstruktur (36), dem festen Duftstoff (3), der Entlüftungsnut (28), dem Entlüftungsrohr (31) und dem inneren Hohlraum des Ansaugstutzens (2) ausgebildet ist.

18. Elektrische Zigarette, bei der die elektrische Zigarette den Zerstäuber für eine elektrische Zigarette nach einem der Ansprüche 1 bis 17 verwendet.

## Revendications

1. Atomiseur de cigarette électronique, comprenant un logement (1) qui est en liaison fixe avec une buse d'aspiration (2), dans lequel une pièce d'atomisation ultrasonique est fixée dans le logement (1) ; la pièce d'atomisation ultrasonique comprend une pièce en céramique piézoélectrique solide (6) ; **caractérisé en ce que**
une surface de la pièce en céramique piézoélectrique solide (6) est en contact avec une structure de guidage de liquide, et l'autre surface de celle-ci est en contact avec un parfum solide (3) ; la structure de guidage de liquide communique avec une cavité de stockage de goudron de tabac (4) ; la structure de guidage de liquide et le parfum solide (3) communiquent avec un passage d'écoulement d'air (5) ; une entrée d'air du passage d'écoulement d'air (5) communique avec l'extérieur du logement (1), et une sortie du passage d'écoulement d'air (5) communique avec la buse d'aspiration (2).

2. Atomiseur de cigarette électronique selon la revendication 1, dans lequel le parfum solide comprend un matériau de cigarette, un extrait de tabac, une essence et un humectant ; et le rapport de poids du matériau de cigarette, de l'extrait de tabac, de l'essence et de l'humectant est 1: 0,1-0,5: 0,001-0,005: 2-6.

3. Atomiseur de cigarette électronique selon la revendication 1, dans lequel le plan où se trouve la pièce en céramique piézoélectrique solide (6) est parallèle à l'axe central du logement (1).

4. Atomiseur de cigarette électronique selon la revendication 3, dans lequel la structure de guidage de liquide comprend un corps de guidage de liquide (8) et une pièce de stockage de liquide (7) qui est en contact avec une surface de la pièce en céramique piézoélectrique solide (6) ; la pièce de stockage de liquide (7) est en contact avec une extrémité du corps de guidage de liquide (8) ; et l'autre extrémité du corps de guidage de liquide (8) s'étend dans la cavité de stockage de liquide (4).

5. Atomiseur de cigarette électronique selon la revendication 4, dans lequel le corps de guidage de liquide (8) comprend une partie de connexion qui a une forme et une taille qui correspondent à celles de la pièce de stockage de liquide (7) et a une position opposée à celle de celle-ci, et une partie de guidage de liquide s'étendant dans la cavité de stockage de liquide (4) et connectée avec la partie de connexion ; et au moins une saillie (9) en contact avec la pièce de stockage de liquide (7) est prévue sur la partie de connexion.

6. Atomiseur de cigarette électronique selon la revendication 5, dans lequel la partie de connexion est fixée dans une base de fixation (10), une encoche (11) est prévue dans la base de fixation (10) et la partie de guidage de liquide s'étend dans la cavité de stockage de liquide (4) depuis l'encoche (11).

7. Atomiseur de cigarette électronique selon la revendication 6, dans lequel la pièce en céramique piézoélectrique solide (6) et la pièce de stockage de liquide (7) sont fixées dans un support (12) ; la base de fixation (10) est reliée à un côté du support (12) qui est proche de la pièce de stockage de liquide (7) ; une base de fixation de parfum (15) est prévue sur un côté du support (12) qui est éloigné de la pièce de stockage de liquide (7) ; une cavité, à savoir une cavité de remplissage (16) est prévue entre la base de fixation de parfum (15) et la pièce en céramique piézoélectrique solide (6), la cavité de remplissage (16) est remplie avec le parfum solide (3) ; un orifice d'entrée d'air (17) et un orifice de sortie d'air (18), qui sont situés dans des positions opposées et communiquent avec le parfum solide (3), sont respectivement prévus dans le support (12) sur le côté supérieur et le côté inférieur de la cavité de remplissage (16) ; et l'orifice de sortie d'air (18) communique avec la buse d'aspiration (2).

8. Atomiseur de cigarette électronique selon la revendication 7, dans lequel une entrée d'air (19) est prévue dans le logement (1) ; un premier espace (20) est prévu entre la pièce de stockage de liquide (7) et le corps de guidage de liquide (8), et un côté du premier espace (20) communique avec l'entrée d'air (19) ; et l'autre côté du premier espace (20) communique avec l'orifice d'entrée d'air (17).

9. Atomiseur de cigarette électronique selon la revendication 8, dans lequel la cavité de stockage de liquide (4) est prévue au bas de la pièce en céramique piézoélectrique solide (6) et le corps de guidage de liquide (8) ; un couvercle de cavité (21) est prévu à l'extrémité supérieure de la cavité de stockage de liquide (4) ; une surface du couvercle de cavité (21) qui est éloignée de la cavité de stockage de liquide (4) est reliée à un bord latéral d'une cavité creuse (22) qui a une extrémité supérieure ouverte, et une marche de limitation (23) est prévue sur la paroi intérieure de la cavité creuse (22) ; la paroi extérieure au bas du support (12) est reliée à la paroi intérieure de la marche de limitation (23) ; un premier orifice traversant (13) est prévu dans une position de contact du support (12) et de la cavité creuse (22) ; le premier espace (20) communique avec l'orifice d'entrée d'air (17) par le premier orifice traversant (13) ; et l'entrée d'air (19), le premier espace (20), le premier orifice traversant (13), l'orifice d'entrée d'air (17), la cavité creuse (22), l'orifice de sortie d'air (18) et la cavité intérieure de la buse d'aspiration (2) forment le passage d'écoulement d'air (5).

10. Atomiseur de cigarette électronique selon la revendication 9, dans lequel un orifice traversant d'installation (24) est prévu dans un côté du couvercle de cavité (21) ; et la partie de guidage de liquide du corps de guidage de liquide (8) s'étend dans la cavité de stockage de liquide (4) après pénétration par l'orifice traversant d'installation (24).

11. Atomiseur de cigarette électronique selon la revendication 9, dans lequel l'extrémité inférieure de la cavité de stockage de liquide (4) est prévue dans un couvercle inférieur (25) ; et la paroi extérieure du couvercle inférieur (25) est en liaison fixe avec la paroi intérieure du logement (1).

12. Atomiseur de cigarette électronique selon la revendication 1, dans lequel la cavité de stockage de liquide (4) est prévue en haut de la pièce en céramique piézoélectrique solide (6), la pièce en céramique piézoélectrique solide (6) est fixée dans une cavité, la cavité est formée en entourant une base de fixation inférieure (27) qui est ouverte aux deux extrémités et une base de fixation supérieure (29) dont l'extrémité inférieure est prévue dans la base de fixation inférieure (27) ; une rainure de ventilation (28) est prévue dans la paroi intérieure de la base de fixation inférieure (27) ; la surface supérieure de la pièce en céramique piézoélectrique solide (6) est en contact avec la structure de guidage liquide ; la surface inférieure de la pièce en céramique piézoélectrique solide (6) est en contact avec le parfum solide (3) ; et la base de fixation inférieure (27) est reliée avec le bas du logement (1).

13. Atomiseur de cigarette électronique selon la revendication 12, dans lequel la structure de guidage de liquide comprend un corps de guidage de liquide (8) ; le corps de guidage de liquide (8) comprend un corps principal qui est en contact avec une surface de la pièce en céramique piézoélectrique solide (6) ; au moins une saillie (9) est prévue sur le corps principal ; et la saillie (9) communique avec la cavité de stockage de liquide (4) après pénétration par la base de fixation supérieure (29).

14. Atomiseur de cigarette électronique selon la revendication 13, dans lequel un tuyau de ventilation (31) est prévu dans la cavité de stockage de liquide (4) ; une partie d'installation (32) est prévue à l'extrémité inférieure du tuyau de ventilation (31) ; un orifice de montage (33) dont le diamètre intérieur correspond au diamètre extérieur de la partie d'installation (32) est prévu dans la base de fixation supérieure (29) ; la partie d'installation (32) est fixée dans l'orifice de montage (33) et la cavité intérieure de la partie d'installation (32) communique avec la rainure de ventilation (28) ; et l'extrémité supérieure du tuyau de ventilation (31) communique avec la cavité intérieure de la buse d'aspiration (2).

15. Atomiseur de cigarette électronique selon la revendication 14, dans lequel la base de fixation inférieure (27) est prévue sur une base de connexion (34) ; un deuxième orifice traversant (35) est prévu dans la base de connexion (34) ; une structure de filet (36) est prévue entre le deuxième orifice traversant (35) et la base de fixation inférieure (27) ; le parfum solide (3) est placé dans la cavité creuse entre la structure de filet (36) et la pièce en céramique piézoélectrique solide (6).

16. Atomiseur de cigarette électronique selon la revendication 15, dans lequel une électrode (37) est installée dans le deuxième orifice traversant (35) ; un passage d'entrée d'air (38) avec une extrémité communiquant avec l'extérieur est prévu dans l'électrode (37) ; et le passage d'entrée d'air (38) communique avec la rainure de ventilation (28) par la structure de filet (36) et le parfum solide (3).

17. Atomiseur de cigarette électronique selon la revendication 16, dans lequel au moins un orifice d'air (39) en communication avec le parfum solide (3) est prévu en haut de l'électrode (37) ; et le passage d'écoulement d'air (5) est formé par le passage d'entrée d'air (38), l'orifice d'air (39), la structure de filet (36), le parfum solide (3), la rainure de ventilation (28), le tuyau de ventilation (31) et la cavité intérieure de la buse d'aspiration (2).

18. Cigarette électronique, dans laquelle la cigarette électronique utilise l'atomiseur de cigarette électronique selon l'une quelconque des revendications 1 à 17.
